# EUROPEAN PATENT SPECIFICATION

(11) **EP 3 375 480 B1**
(45) Date of publication and mention of the grant of the patent: **30.10.2019**
(21) Application number: 17160534.8
(22) Date of filing: 13.03.2017
(51) Int. Cl.: A61M 25/02

(54) **SUTURE CLIP**
NAHTKLAMMER
PINCE DE SUTURE

(43) Date of publication of application: 19.09.2018
(73) Proprietor: Contract Medical International GmbH, 01277 Dresden (DE)
(72) Inventor: MATTHES, Martin, 01809 Heidenau (DE); WIESNER, Thomas, 01454 Wachau (DE); HURTAK, Wenzel, F-83310 Cogolin (FR)
(74) Representative: Simandi, Claus

(56) References cited:
- CN-A- 104 984 428
- FR-A1- 2 643 269
- GB-A- 2 422 553
- US-A1- 2010 145 280

## Description

The present invention relates to fixation accessories, so called clips, specifically designed for holding medical devices, such as catheters, cables or tubing to an animal or human body, for example after or during surgery or medical treatment. The invention provides such a clip in the form of a hinged clamp that holds the medical device, the clamp comprising two jaw elements connected via a bending joint to form a central clamping aperture for clamping the medical device.

Known fixation or retaining devices for medical use engage with a medical device, such as tubing, catheters, needles or other devices of basically cylindrical or tubular shape, extending from or entering the animal or human body in order to hold and secure that medical device to the outer skin of said body. Generally, such fixation devices are attached to the skin of the animal or human body by glue pads and/or by individual sutures.

Disadvantageously, such fixation devices lack good handling and thus have limited usability. For example, the devices, once engaged to hold the medical device, cannot be easily re-opened and, in particular, the medical device re-arranged. This, however, is of particular importance, if, in the course of the medical treatment or directly during operation the medical device has to be exchanged or re-positioned with the fixation device already fixed in place on the body.

Yet another problem of known fixation devices is the lack of flexibility with regard to the shape and nature of the medical device to be fixated within the device. Known fixation devices have to be closely adapted to the particular medical device to be fixated. Moreover, known fixation devices are unable to perform further medical functions beyond the rather simple fixation task, for example, compressing or closing or releasing a held medical device such as a catheter or tubing, in particular when the fixation device is already fixed in place on the body. FR 2 643 269 A, GB 2 422 553 A, and US 2010/145280 A1 disclose hinged clamps for holding a medical device comprising jaw elements that are coupled at a joint and form hinge and a clamping aperture between the jaw elements.

The present invention provides a new and improved clamp for holding a medical device which fully overcomes these issues and technical problem and thus provides a flexible and easy-to-handle fixation device with improved and extended usability, in particular in the medical field for prophylactic and therapeutic treatment. The clamp of the present invention specifically allows for exerting controlled forces between different medical devices. The clamp allows for forcing changes in the dimensional shape of the medical devices in a controlled manner. It allows for easy release of the device and re-fixation possibility without the need of undoing the suture.

The invention provides a hinged clamp according to claim 1, comprising a hinge and a central clamping aperture, the clamp being specifically designed for holding a medical device, and/or for use with a medical device, the clamp comprising a first jaw element having two handles, which each extend to opposite ends of this jaw element and a second jaw element also having two handles also each extending to opposite ends of this second jaw element. Both jaw elements are coupled together via a common bending joint, which forms the hinge between the two jaws, by means of inherent elasticity of the material used for the joint. The hinged first and second jaw element, together with the bending joint, form a central clamping aperture, with a lateral opening opposite the bending joint for clamping the medical device. The two jaws and the bending joint together are preferably formed on the same piece and the thus formed hinged clamp is one-pieced.

According to the invention, at the so-called "hinge-side" of the clamp, each one of the hinge-sided handles of the two jaw elements is arranged in a general side-by-side configuration or fashion to the other handle to allow for manual action to effect opening of the central clamping aperture, for example by pinching or pressing the two-opposing hinge-sided handles together.

That is, the present invention particularly provides: A hinged clamp for holding a medical device, comprising: a first jaw element (32), comprising at least one first hinge-sided handle (22) extending to one end of this element; a second jaw element (36), comprising at least one second hinge-sided handle (26) extending to one end of this element; and a bending joint (30) coupling said first jaw element (32) and said second jaw element (36) to form a hinge and a central clamping aperture (40) with a lateral opening (48) opposing the joint (30) for clamping the medical device; wherein on the hinge-side (12) of the clamp said hinge-sided handles (22,26) are arranged generally in a side-by-side configuration for manual action to effect opening of said central clamping aperture (40).

The two hinge-sided handles according to the present invention allow for easy and convenient manual operation of the hinged clamp to effect said release of the medical device from the central clamping aperture. These handles allow for safe and secure application of such clamp opening forces. Advantageously, due to the side-by-side, i.e. opposing configuration of both hinge-sided handles, any manual action to effect opening of the clamping aperture does not result in any unfavorable, detrimental or even harmful net forces which would adversely move the fixation clamp as a whole from its predetermined position on the body. The hinge-sided handles of the clamp are designed and arranged such, that the manually applied forces to open the clamp are balanced. This also supports a secure grip of the handles themselves by the manual operator. By that, and in contrast to known fixation devices, a clamping of the medical device in the essential clamping aperture can be loosened and the medical device released, re-arranged, re-positioned or removed or exchanged without the need of removing or altering the position of the hinged clamp from the animal or human body once attached to it. Further, there is no need for a specific separate opening or release tool, and the clamp can be operated not only by the medical operator during surgery or therapy, but also by trained and even untrained medical personnel, without the risk of adverse or even harmful effects to the transdermal opening of the body, the clamp is attached to.

The hinged clamp of the present invention further comprises two handles on the so-called "clamp-side" of the clamp, which are preferably also arranged in a side-by-side configuration analog to the arrangement of the hinge-sided handles. By that, the clamp-sided handles allow for manual action to effect closing or further closing of the central clamping aperture. By that, it is possible to either clamp a medical device which otherwise would be in a loose fit within the central aperture of the clamp or to exert additional force to the medical device held within the central aperture, for example, and preferably, to compress or even close the medical device temporarily or permanently.

For a permanent increase of the closing forces, i.e. further closing of the central aperture, for example to compress or close the medical device, according to the invention, the clamp includes a detent on one of the clamp-sided handles, which releasably engages a specific engaging member on the other, opposing clamp-sided handle. By that, a permanent, but releasable further closing of the central clamping aperture is possible, if needed, just by manually pressing the two clamp-sided handles together until the detent is engaged.

In a preferred embodiment thereof, the clamp-sided handle having the detent further includes a curved section which functions as an elastic element or bending joint to allow for better engaging and release of the detent. In a preferred variant thereof, this handle further comprises a distal lever section to allow for easy manual action to effect release of the engagement of detent and engaging member.

The hinged clamp described herein also allows to easily secure a second medical device, such as a needle or probe, which is passed through the first medical device, which, for example, is a tubing or sleeve, and to fixate or release the second to the first one, by use of the herein described controlled squeezing or pinching or by controlled release by manual operation of the two clamp-side handles or the two hinge-side handles, respectively. This allows for an effective but indirect fixation of the second device also to the predetermined position
In a preferred embodiment, the central clamping aperture formed within the hinged clamp forms a basically or specifically cylindrical hub. This hub is lined with a bushing which preferably is comprised of a separate soft material. In a preferred embodiment thereof, the cylindrical hub has a fixed diameter and the bushing is variable with different inner diameters. The inner diameter of the bushing is specifically adapted to the form and dimension of the medical device to be fixated within the clamp. By that, the present invention provides a general basic body of hinged clamp to which such variable or specifically adapted bushings can be fitted. The basic body of the clamp can be produced as a preferably one-pieced product to which variable, specifically adapted bushings can be fitted, thus reducing production costs and even allowing re-usability of one or more of the parts of the clamp. Thus, in a preferred embodiment, the inner bushing is separate and preferably releasable from the cylindrical hub of the central aperture. Clamp and bushing may be formed in separate processes to finally result in a two-pieced product. In an alternative embodiment, the bushing is firmly and permanently connected to the cylindrical hub. Bushing and clamp may be formed in a one- or two-step process to finally result in a one-pieced product.

In a preferred variant, the bushing has different elasticity than the base material of the clamp. Preferably, the bushing is formed from an elastomer material which has higher elasticity. The bushing can be made from medical compatible elastomers, co-polymers thereof and foams thereof. Preferably the soft material of the bushing is selected from one of: PU-based elastomers, PE-based elastomers, PP-based elastomers, PP/EPDM, PA-based elastomers, silicones, latex, as well as co-polymers and/or foams thereof.

In a preferred embodiment, the soft material of the bushing has Shore A-hardness of 20 to 50. In another preferred variant, the soft material bushing is a yielding soft material with some elasticity but higher resiliency. Soft materials better adapt to the outer shape and dimension of the medical device and allow, in particular, for better fixation of the medical device within the central aperture.

Even more, while clamping may be released or loosened by releasing the engagement of the two clamp-sided handles, the medical device may still remain in place on the clamp of the present invention, due to a combination of sizing and high friction coefficient of the soft material bushing. Gently pressing the hinge-sided handles, allows for controlled displacing or sliding of the clip to a selected new position on the medical device.

Within the context of the present invention, a "medical device" is any, preferably cylindrical or tubular shaped device to be inserted into or extending from the animal or human body, preferably at a transdermal opening. A medical device according to the present invention is preferably selected from one of: tubing, catheters, cables, introducer sheaths, needles, and cylindrical or tubular sensors. A typical medical application is the fixation of a catheter which enters the animal or human body at the site of the clamp with the clamp itself held or fixed in place on the body.

The clamp further comprises at least one, preferably at least two or more lateral eyelets at one or more of the four handles which allow for suturing or clamping the clamp of the invention to the animal or human body, preferably to the outer skin.

Also described but not forming part of the present invention is a method or the use of the clamp in medical treatment, therapy or diagnosis of an animal or human body, preferably for use together with a medical device such as a transdermal catheter, tubing or needle.

Also described but nor forming part of the present invention is a method or the use of the clamp for securing a medical device to the skin or other tissue of an animal or human body. The hinged clamp described herein allows for fixation possibilities to other tissue than skin. For instance, at intraoperative use, attaching the clip to muscle or other structured tissue inside the body or in the proximity of an organ.

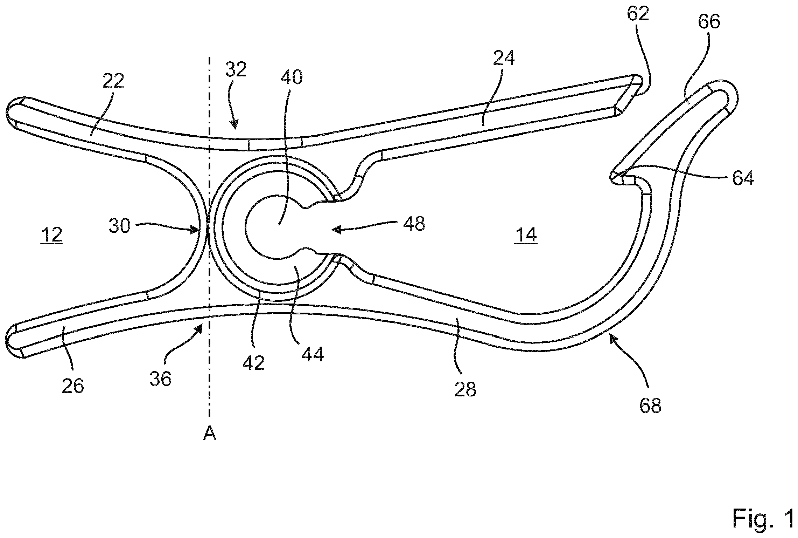

A fourth aspect of the present invention is a kit of parts for medical use, comprising at least one medical device and at least one clamp of the present invention to fixate this medical device. The invention is further described on a particular embodiment, which is considered to be non-limiting to the present invention. Figure 1 is a plan view (not to scale) of one embodiment of the clamp: Handles (22) and (24) form a first jaw element (32). This jaw element is coupled to a second jaw element (36), formed by handles (26) and (28) via a bending joint (30) which serves as the hinge of the clamp. First jaw element (32), second jaw element (36) and bending joint (30) are arranged such that a central aperture (40) is formed in between said elements (30, 32, 36). The central aperture (40) has lateral opening (48). Opening (48) allows for introducing a medical device into the clamp's aperture (40) and, secondly, allows for hinging action at the bending joint (30) on the opposite side of the aperture (40). The central aperture (40) comprises a cylindrical hub (42) with a bushing (44) of separate and preferably softer material. For illustrative purposes, a line "A" can be drawn to separate a hinge-side (12) of the clamp from a clamp-side (14) of the clamp. On the hinge side (12) handles (22) and (26) of first and second jaw element (32, 36) are arranged in a side-by-side configuration, i.e. opposing each other. Any manual action, in particular pinching and pressing the two handles (22, 26) together, results in an opening of the aperture (40). On the clamp side (14) jaws (24) and (28) are arranged in a similar side-by-side configuration. Any manual action to these handles leads to a pinching or closing of the aperture (40). In the depicted preferred embodiment, handle (28) further has a detent (64) to engage with engaging member (62) of the other handle (24). A releasable engagement of the two handles (24, 28) is facilitated by the provision of a curved section (68) on handle (28) which serves as an elastic element and by a lever element (66), which allows for manual action to release the detent (64) from the engaging member (62).

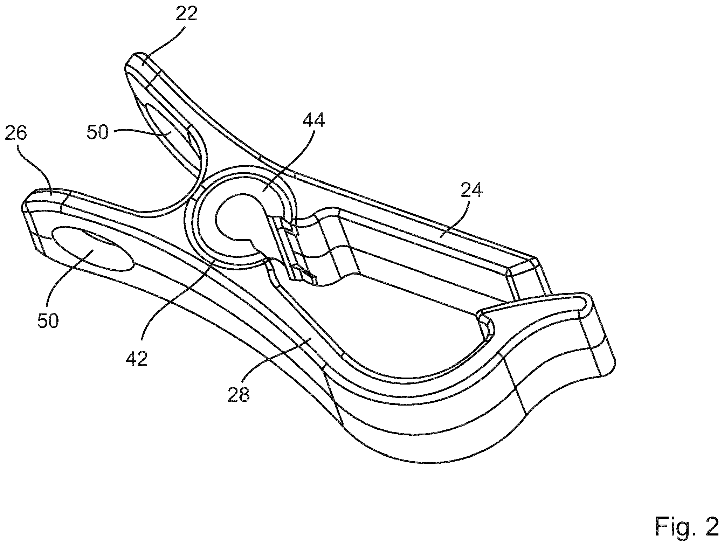

Figure 2 is a perspective view (not to scale) of the clamp of figure 1. Additionally, lateral eyelets (50) for sutures are depicted. The eyelets (50) are preferably present at least in the hinge-side handles (22, 26).

### REFERENCE LIST

- 12: hinge side of the clamp
- 14: clip side of the clamp
- 22: first hinge-side handle
- 26: second hinge-side handle
- 24: first clamp-side handle
- 28: second clamp-side handle
- 30: bending joint
- 32: first jaw element
- 34: second jaw element
- 40: central aperture
- 42: cylindrical hub
- 44: bushing
- 48: lateral opening
- 50: eyelet
- 62: engaging member
- 64: detent
- 66: lever element
- 68: curved section

## Claims

1. A hinged clamp with a hinge (30) and a central clamping aperture (40) for holding a medical device, comprising:
a first jaw element (32), comprising at least one first hinge-sided handle (22) on the side of the hinge (30) extending to one end of this element;
a second jaw element (36), comprising at least one second hinge-sided handle (26) on the side of the hinge (30) extending to one end of this element; and
a bending joint (30) coupling said first jaw element (32) and said second jaw element (36) to form the hinge (30) and the central clamping aperture (40) with a lateral opening (48) opposing the joint (30) for clamping the medical device;
wherein on the hinge-side (12) of the clamp said hinge-sided handles (22,26) are arranged opposing each other in a side-by-side configuration for manual action to effect opening of said central clamping aperture (40); **characterized in that** on the clamp side (14) of the clamping aperture (40) opposite to the hinge side (12) each jaw element (32,36) further comprises a clamp-sided handle (24,28) said clamp-sided handles (24,28) are arranged opposing each other in a side-by-side configuration for manual action to effect further closing of said central clamping aperture (40).

2. The clamp of claim 1, wherein one of said clamp-sided handle (28) forms a releasable detent (64) to engage with an engaging member (62) formed on the other said clamp-sided handle (24) to effect permanent further closing of said central clamping aperture (40).

3. The clamp of claim 2, wherein said one clamp-sided handle (28) has a curved section (68) that forms a second bending joint on said handle (28).

4. The clamp of claim 3, wherein said one clamp-sided handle (28) further has a jaw section (66) for manual action to effect release of said releasable detent (64) when engaged with engaging member (62) of the said other clamp-sided handle (24) .

5. The clamp of one of preceding claims, wherein the central clamping aperture (40) forms a cylindrical hub (42), which is lined with a bushing (44) of a separate soft material.

6. The clamp of claim 5, wherein the soft material of the bushing (44) is a medically acceptable polymer, selected from one of: PU-based elastomers, PE-based elastomers, PP-based elastomers, PP/EPDM, PA-based elastomers, silicones, latex, co-polymers and foams thereof.

7. The clamp of claim 5 or 6, wherein the soft material of the bushing (44) has a Shore A- hardness of 40 to 60.

8. The clamp of one of the preceding claims, wherein one or more of said handles (22,24,26,28) has one or more lateral eyelets (50) for suturing.

9. The clamp of one of the preceding claims, wherein the medical device is selected from one of: tubing, catheters, cables, introducer sheaths, needles, and cylindrical or tubular sensors.

10. The clamp of one of the preceding claims for use in medical treatment of an animal or human body.

11. The clamp of one of the preceding claims for use in securing a medical device to the skin or other tissue of an animal or human body.

12. A kit of parts for medical use comprising: a medical device and at least one clamp of one of claims 1 to 9 for holding the medical device.

## Patentansprüche

1. Scharnierklemme mit einem Scharnier (30) und einer zentralen Klemmöffnung (40) zum Halten einer medizinischen Vorrichtung, umfassend:
ein erstes Klemmbackenelement (32), das mindestens einen ersten scharnierseitigen Griff (22) auf der Seite des Scharniers (30) aufweist, der sich zu einem Ende dieses Elements hin erstreckt;
ein zweites Klemmbackenelement (36), das mindestens einen zweiten scharnierseitigen Griff (26) auf der Seite des Scharniers (30) aufweist, der sich zu einem Ende dieses Elements hin erstreckt;
ein Biegegelenk (30), welches das erste Klemmbackenelement (32) und das zweite Klemmbackenelement (36) koppelt, um das Scharnier (30) und die zentrale Klemmöffnung (40) mit einer lateralen Öffnung (48), die dem Gelenk (30) gegenüberliegt, um die medizinische Vorrichtung einzuspannen, auszubilden;
wobei auf der Scharnierseite (12) der Klemme die scharnierseitigen Griffe (22, 26) einander in einer Nebeneinanderkonfiguration gegenüberliegend für eine manuelle Betätigung angeordnet sind, um eine Öffnung der zentralen Klemmöffnung (40) zu bewirken; **dadurch gekennzeichnet, dass**
auf der Klemmenseite (14) der Klemmöffnung (40) entgegengesetzt zur Scharnierseite (12) jedes Klemmbackenelement (32, 36) ferner einen klemmenseitigen Griff (24, 28) umfasst, wobei die klemmenseitigen Griffe (24, 28) einander in einer Nebeneinanderkonfiguration gegenüberliegend für eine manuelle Betätigung angeordnet sind, um eine weitere Schließung der zentralen Klemmöffnung (40) zu bewirken.

2. Klemme nach Anspruch 1, wobei einer der klemmenseitigen Griffe (28) eine lösbare Arretierung (64) bildet, die mit einem Eingriffselement (62), das am anderen klemmenseitigen Griff (24) ausgebildet ist, in Eingriff zu bringen ist, um eine permanente weitere Schließung der zentralen Klemmöffnung (40) zu bewirken.

3. Klemme nach Anspruch 2, wobei der eine klemmenseitige Griff (28) einen gekrümmten Abschnitt (68) aufweist, der ein zweites Biegegelenk am Griff (28) bildet.

4. Klemme nach Anspruch 3, wobei der eine klemmenseitige Griff (28) ferner einen Klemmbackenabschnitt (66) für eine manuelle Betätigung aufweist, um eine Lösung der lösbaren Arretierung (64) zu bewirken, wenn er mit einem Eingriffselement (62) des anderen klemmenseitigen Griffs (24) in Eingriff kommt.

5. Klemme nach einem der vorangehenden Ansprüche, wobei die zentrale Klemmöffnung (40) eine zylindrische Nabe (42) bildet, die mit einer Hülse (44) aus einem separaten weichen Material ausgekleidet ist.

6. Klemme nach Anspruch 5, wobei das weiche Material der Hülse (44) ein medizinisch annehmbares Polymer ist, ausgewählt aus einem von: Elastomeren auf PU-Basis, Elastomeren auf PE-Basis, Elastomeren auf PP-Basis, PP/EPDM, Elastomeren auf PA-Basis, Silikonen, Latex, Copolymeren und Schäumen davon.

7. Klemme nach Anspruch 5 oder 6, wobei das weiche Material der Buchse (44) eine Shore-A-Härte von 40 bis 60 aufweist.

8. Klemme nach einem der vorangehenden Ansprüche, wobei einer oder mehrere von den Griffen (22, 24, 26, 28) eine oder mehrere laterale Ösen (50) für eine Nahtbildung aufweist.

9. Klemme nach einem der vorangehenden Ansprüche, wobei die medizinische Vorrichtung ausgewählt ist aus einem von:
Schläuchen, Kathetern, Kabeln, Einführhülsen, Nadeln und zylindrischen oder röhrenförmigen Sensoren.

10. Klemme nach einem der vorangehenden Ansprüche zur Verwendung bei einer medizinischen Behandlung eines Tieres oder menschlichen Körpers.

11. Klemme nach einem der vorangehenden Ansprüche zur Verwendung bei der Sicherung einer medizinischen Vorrichtung an der Haut oder einem anderen Gewebe eines Tieres oder menschlichen Körpers.

12. Kit aus Teilen für eine medizinische Verwendung, umfassend: eine medizinische Vorrichtung und mindestens eine Klemme nach einem der Ansprüche 1 bis 9 zum Halten der medizinischen Vorrichtung.

## Revendications

1. Pince articulée dotée d'une charnière (30) et d'une
ouverture de serrage centrale (40) pour tenir un dispositif médical, comprenant :
un premier élément de mâchoire (32), comprenant au moins une première poignée côté charnière (22) sur le côté de la charnière (30) s'étendant jusqu'à une extrémité de cet élément ;
un second élément de mâchoire (36), comprenant au moins une seconde poignée côté charnière (26) sur le côté de la charnière (30) s'étendant jusqu'à une extrémité de cet élément ; et
une articulation flexible (30) couplant ledit premier élément de mâchoire (32) et ledit second élément de mâchoire (36) afin de former la charnière (30) et l'ouverture de serrage centrale (40) avec une ouverture latérale (48) à l'opposé de l'articulation (30) pour serrer le dispositif médical ;
où lesdites poignées côté charnière (22, 26) sont agencées sur le côté pince (12) de la pince opposées l'une à l'autre dans une configuration côte à côte pour une action manuelle afin d'effectuer l'ouverture de ladite ouverture de serrage centrale (40) ; **caractérisée en ce que**, sur le côté pince (14) de l'ouverture de serrage (40), à l'opposé du côté charnière (12), chaque élément de mâchoire (32, 36) comprend en outre une poignée côté pince (24, 28), lesdites poignées côté pince (24, 28) sont agencées opposées l'une à l'autre dans une configuration côte à côte pour une action manuelle afin d'effectuer l'ouverture de ladite ouverture de serrage centrale (40) .

2. Pince selon la revendication 1, dans laquelle une desdites poignées côté pince (28) forme une détente libérable (64) pour s'engager avec un élément d'engagement (62) formé sur l'autre poignée côté pince (24) afin d'effectuer une nouvelle fermeture permanente de ladite ouverture de serrage centrale (40).

3. Pince selon la revendication 2, dans laquelle ladite poignée côté pince (28) a une section (68) courbe qui forme une deuxième articulation flexible sur ladite poignée (28).

4. Pince selon la revendication 3, dans laquelle ladite poignée côté pince (28) a en outre une section de mâchoire (66) pour une action manuelle afin d'effectuer une libération de ladite détente libérable (64) lorsqu'elle est engagée avec l'élément d'engagement (62) de ladite autre poignée côté pince (24).

5. Pince selon l'une des revendications précédentes, dans laquelle l'ouverture de serrage centrale (40) forme un pivot (42) cylindrique qui est doublé d'un manchon (44) dans une matière souple séparée.

6. Pince selon la revendication 5, dans laquelle la matière souple du manchon (44) est un polymère acceptable au niveau médical choisi parmi l'un des polymères : des élastomères à base de PU, des élastomères à base de PE, des élastomères à base de PP, du PP/EPDM, des élastomères à base de PA, des silicones, du latex, des copolymères et des mousses de ceux-ci.

7. Pince selon la revendication 5 ou la revendication 6, dans laquelle la matière souple du manchon (44) a une dureté Shore A de 40 à 60.

8. Pince selon l'une des revendications précédentes, dans laquelle une ou plusieurs desdites poignées (22, 24, 26, 28) a un ou plusieurs œillets latéraux (50) pour suturer.

9. Pince selon l'une des revendications précédentes, dans laquelle le dispositif médical est choisi parmi : des tubes, des cathéters, des câbles, des gaines d'introduction, des aiguilles et des capteurs cylindriques ou tubulaires.

10. Pince selon l'une des revendications précédentes destinée à une utilisation dans le traitement médical d'un corps animal ou humain.

11. Pince selon l'une des revendications précédentes destinée à une utilisation dans la fixation d'un dispositif médical sur la peau ou un autre tissu d'un corps animal ou humain.

12. Ensemble de pièces pour une utilisation médicale comprenant :
un dispositif médical et au moins une pince selon l'une des revendications 1 à 9 pour maintenir le dispositif médical.
